# EUROPÄISCHE PATENTANMELDUNG

(11) **EP 2 730 220 A1**
(43) Veröffentlichungstag der Anmeldung: **14.05.2014**
(21) Anmeldenummer: 12192223.1
(22) Anmeldetag: 12.11.2012
(51) Int. Cl.: A61B 5/097

(54) **Vorrichtung zur Vortrocknung eines Gasstroms, sowie Gasprobenleitung und Gasanalysator mit derselben**

(71) Anmelder: Bluepoint Medical GmbH & Co. KG, 23923 Selmsdorf (DE)
(72) Erfinder: Lindner, Bernd, 23167 Stockelstorf (DE)
(74) Vertreter: Seemann, Ralph

(57) **Zusammenfassung**

Die Erfindung betrifft eine Vorrichtung (10, 110) zur Vortrocknung eines Gasstroms mit einer zentralen Bohrung (12) mit einem Einlass und einem Auslass zur Durchleitung eines vorzutrocknenden Gasstroms, insbesondere eines Messgasstroms. Die Erfindung betrifft ferner eine Gasprobenleitung, umfassend wenigstens einen Schlauch zur Leitung eines zu analysierenden Gases, insbesondere Atemluft, sowie einen Gasanalysator, insbesondere Kapnograph.

Die erfindungsgemäße Vorrichtung (10, 110) weist wenigstens eine Kapillare zur Abführung von Feuchtigkeit aus dem Gasstrom auf, die mit der zentralen Bohrung (12) einerseits und mit einer Wand aus einem Material mit hoher Wasserdampftransportrate andererseits in Kontakt steht.

## Beschreibung

Die Erfindung betrifft eine Vorrichtung zur Vortrocknung eines Gasstroms mit einer zentralen Bohrung mit einem Einlass und einem Auslass zur Durchleitung eines vorzutrocknenden Gasstroms, insbesondere eines Messgasstroms. Die Erfindung betrifft ferner eine Gasprobenleitung, umfassend wenigstens einen Schlauch zur Leitung eines zu analysierenden Gases, insbesondere Atemluft, sowie einen Gasanalysator, insbesondere Kapnograph.

Messgasanalysatoren wie beispielsweise Kapnographen werden im klinischen Umfeld eingesetzt, um stationär behandelte oder beatmete Patienten zu überwachen. Dabei wird der Gehalt an Kohlenstoffdioxid in der Ausatemluft eines Patienten gemessen und überwacht. Die Überwachung sorgt für eine deutliche Reduktion von Komplikationen insbesondere bei beatmeten Patienten. Auch in der Anästhesie werden sie bei Einsatz von Narkosegeräten eingesetzt.

Auch andere Gase, wie beispielsweise Sauerstoff, Lachgas oder gasförmige Narkosemittel können in diesem Zusammenhang überwacht werden.

Je nach Messtechnik unterscheidet man Hauptstromverfahren und Nebenstrom- bzw. Seitenstromverfahren. Beim Seitenstromverfahren wird eine geringe Menge Luft ständig abgesaugt und über einen dünnen Schlauch als Gasprobenleitung zum Gasanalysator geleitet. Dabei erfolgt die Messung verzögert. Die Schlauchlängen betragen in diesem Fall typischerweise etwa bis zu 2 bis 3 Meter.

Ein Umstand, der die Überwachung erschwert, ist die in der Atemluft enthaltene Feuchtigkeit. Diese schlägt sich als Kondensat an den Wänden des Schlauches mit dem Messgasstrom nieder und muss vom Gasanalysator ferngehalten werden, um diesen nicht zu beschädigen. Hierfür werden insbesondere Wasserfallen eingesetzt, die Kondenswasser auffangen und in einem mit dem Schlauch verbundenen Reservoir speichern. Wenn dieses Volumen bzw. Reservoir aufgefüllt ist, muss die Wasserfalle entleert oder ersetzt werden.

Insbesondere bei einem Nebenstromverfahren oder Seitenstromverfahren ist es außerdem wichtig, dass ein geringes Totvolumen in dem Nebenstromschlauch bzw. der Gasprobenleitung vorhanden ist, was die Überwachung verschlechtern würde. Ein großes Totvolumen führt bei gegebener Absaugrate zu Mischluft aus inspiratorischer und respiratorischer Phase und damit zu einer fehlerhaften Messung.

Hiervon ausgehend liegt der vorliegenden Erfindung die Aufgabe zugrunde, eine Gasprobenleitung und gegebenenfalls die Wasserfalle länger benutzbar zu machen als im Stand der Technik möglich.

Diese Aufgabe wird durch eine Vorrichtung zur Vortrocknung eines Gasstroms mit einer zentralen Bohrung mit einem Einlass und einem Auslass zur Durchleitung eines vorzutrocknenden Gasstroms, insbesondere eines Messgasstroms, gelöst, indem die Vorrichtung wenigstens eine Kapillare zur Abführung von Feuchtigkeit aus dem Gasstrom aufweist, die mit der zentralen Bohrung einerseits und mit einer Wand aus einem Material mit hoher Wasserdampftransportrate andererseits in Kontakt steht.

Die Erfindung beruht auf dem Grundgedanken, dass eine Vorrichtung zur Vortrocknung eines Gasstroms in eine Gasprobenleitung integriert wird, bei der Wassertröpfchen, die aus der Atemluft an der Gasprobenleitungswand kondensiert sind, durch einen Kapillareffekt durch eine oder mehrere Kapillare vom Gasstrom weg nach außen abgeführt werden. Der Durchmesser oder die Breite solcher Kapillaren beträgt Bruchteile von Millimetern. Die durch Kapillarkräfte abgeführte Feuchtigkeit gelangt dann zu einer Wand aus einem Material mit hoher Wasserdampftransportrate. Ein solches Material leitet gezielt Wasser nach außen, wo es in der Außenluft bzw. Umgebungsluft aufgenommen wird.

Es ergibt sich ein ständiger Strom von Wasser und Wasserdampf aus dem Gasstrom durch die Kapillare und die Wand aus Material mit hoher Wasserdampftransportrate in die Umgebungsluft. Auf diese Weise wird das aus dem Gasstrom entnommene Wasser komplett entfernt und wird nicht in einem Reservoir aufgefangen, das im Laufe der Zeit vollläuft. Mit der erfindungsgemäßen Vorrichtung lässt sich somit eine Überwachung eines Messgasstroms, beispielsweise im Rahmen einer Kapnographie, wesentlich länger betreiben als mit herkömmlichen Gasprobenleitungen ohne die erfindungsgemäße Vorrichtung.

Da die Vorrichtung durch eine zentrale Bohrung durchsetzt ist, von der nur Kapillaren abzweigen bzw. ein kapillarer Ringspalt, wird der Gasstrom in der zentralen Bohrung nicht gestört. Die Vorrichtung weist kein das Messergebnis verfälschendes Totvolumen auf. Das Volumen außerhalb der zentralen Bohrung wird nicht durch den Gasstrom durchströmt, sondern befindet sich nur mittels Kapillarkräften und einer geringen Mengen von Gasdiffusion im Austausch mit der zentralen Bohrung. Die Gasdynamik in der Kapillare ist eingeschränkt, so dass sich die Gasmoleküle in der Kapillare nicht frei mit der Umgebung, d. h. dem Gasstrom, austauschen können.

Vorzugsweise weist die wenigstens eine Kapillare eine Wandung aus dem Material mit hoher Wasserdampftransportrate auf und/oder besteht die Vorrichtung in wesentlichen Teilen aus dem Material mit hoher Wasserdampftransportrate. Die Transportrate von Wasser bzw. Wasserdampf durch das Material mit hoher Wasserdampftransportrate hängt neben dem Material auch von der zur Verfügung stehenden Oberfläche sowie der Wanddicke ab. Je größer die zur Verfügung stehende Oberfläche ist, desto mehr Wasser bzw. Wasserdampf kann pro Zeiteinheit nach außen in die Umgebungsluft ausgetragen werden. Insofern ist es günstig, bereits auch die Kapillare und oder wesentliche Teile der Vorrichtung aus dem entsprechenden Material zu fertigen. Dies hat außerdem fertigungstechnische Vorteile, da verschiedene Teile im Spritzgussverfahren herstellbar sind und die Anzahl der Teile reduziert werden kann.

Vorzugsweise umfasst das Material mit hoher Wasserdampftransportrate ein sulfoniertes Tetrafluorethylen-Polymer, ein Polyether-block-amid Copolymer oder ein Polyether-ester Blockcopolymer. Das erstgenannte Material ist beispielsweise unter dem Markennamen Nafion^{®} von Du Pont erhältlich. Dieses eignet sich aufgrund hervorragender Wasserdampftransportraten, wobei hierbei nur einfachere geometrische Strukturen wie Folien oder Röhren herstellbar sind. Polyether-block-amide sind beispielsweise unter dem Markennamen Pebax^{®} von Arkema erhältlich, das sehr gute mechanische Eigenschaften aufweist und in beliebige Formen gießbar ist. Das Gleiche gilt für Polyether-ester Blockcopolymere, beispielsweise erhältlich unter dem Markennamen Hytrel^{®} von Du Pont, das sich ähnlich gut verarbeiten lässt wie die Polyether-block-amide. Vergleichbare Produkte sind auch von anderen Herstellern erhältlich. Die letztgenannten Materialien sind bei etwas geringerer Wasserdampftransportrate deutlich kostengünstiger als das sulfonierte Tetrafluorethylen-Polymer.

In einer vorteilhaften Ausgestaltung ist die Kapillare als ringförmiger Kapillarspalt ausgebildet, der insbesondere eine Ebene aufspannt, die senkrecht auf die zentrale Bohrung steht. Ein ringförmiger Kapillarspalt umgibt den Gasstrom vollumfänglich, so dass kondensiertes Wasser an jeder Stelle des Innenumfangs der Gasprobenleitung durch Kapillarkräfte abgeführt wird. Damit steht den Wassertröpfchen eine vollumfängliche Barriere entgegen, die sie an einem Weiterrinnen innerhalb der Gasprobenleitung hindert.

In einer besonders bevorzugten Ausgestaltung der Erfindung oder der erfindungsgemäßen Vorrichtung weist die Vorrichtung einen einlassseitig angeordneten ersten Flächenkörper und einen auslassseitig angeordneten zweiten Flächenkörper auf, deren zueinander weisende Grundflächen zueinander kongruent, insbesondere kreisförmig, ausgebildet sind, wobei die Flächenkörper im zusammengesetzten Zustand jeweils am Umfang nach außen abdichtend miteinander kraftschlüssig, materialschlüssig und/oder formschlüssig verbunden sind, wobei zwischen den Grundflächen ein Spalt verbleibt. Dieser verbleibende Spalt kann den Kapillarspalt darstellen oder beinhalten und/oder einen ringförmigen Raum um die Kapillare herum, der mit einer großen Oberfläche von dem Material mit hoher Wasserdampftransportrate bewandet ist.

In vorteilhaften Ausführungen weist der erste Flächenkörper in seinem zentralen Bereich und/oder der zweite Flächenkörper in seinem zentralen Bereich eine Aussparung auf, wobei in diese Aussparung oder Aussparungen ein erster und/oder zweiter Anschlusskörper eingreift oder eingreifen. So lässt sich der Flächenkörper als einfacher Flächenkörper mit wenig Kontur herstellen, während kompliziertere geometrische Strukturen von dem jeweiligen Anschlusskörper dargestellt sind.

Alternativ geht oder gehen der erste Flächenkörper und/oder der zweite Flächenkörper im zentralen Bereich in einen ersten und/oder zweiten Anschlusskörper über. Somit können erfindungsgemäß der erste Flächenkörper und der erste Anschlusskörper einerseits und der zweite Flächenkörper und der zweite Anschlusskörper andererseits jeweils entweder getrennt oder einstückig ausgebildet sein.

In einer besonders bevorzugten Weiterbildung weist der zweite Flächenkörper mehrere radial nach außen gerichtet angeordnete Abfuhrvolumen auf, die über den Ringspalt bzw. Kapillarspalt oder jeweils eigene Kapillaren mit der zentralen Bohrung verbunden sind, wobei die Abfuhrvolumen bei kleinem Volumen eine große Oberfläche aus Material mit hoher Wasserdampftransportrate aufweisen. Diese nach außen gerichtet angeordneten Abfuhrvolumen ähneln Kühlrippen, indem diese radial nach außen gerichtete, also im Wesentlichen sternförmige, Konfiguration aufweisen. In diesem Fall handelt es sich um Abdampfrippen, die eine große Oberfläche bei kleinem inneren Volumen aufweisen. In dem inneren Volumen befindet sich das durch Kapillarkräfte abgeleitete Wasser sowie ein geringer Anteil an Gas aus dem Messgasstrom, das sich über eine große Oberfläche innerhalb der Abfuhrrippen bzw. Abfuhrvolumen verteilt, das aus einem Material mit hoher Wasserdampftransportrate besteht. In der Variante, in der die Abfuhrvolumen über den Ringspalt mit dem Gasstrom verbunden sind, hat der Ringspalt in diesem Bereich eine Spaltbreite, die einen Kapillareffekt erzeugt.

In einer vorteilhaften und gießtechnisch einfach zu realisierenden Ausgestaltung der erfindungsgemäßen Vorrichtung ist vorgesehen, dass zwischen dem ersten Flächenkörper und/oder Anschlusskörper und dem zweiten Flächenkörper und/oder Anschlusskörper ein Hülsenkörper mit einer zentralen Bohrung angeordnet ist, der einen zylindrischen Teil und an einem Ende des zylindrischen Teils einen scheibenförmigen Teil aufweist, der im zusammengesetzten Zustand der Vorrichtung mit einer Auflagefläche auf der Grundfläche des zweiten Flächenkörpers und/oder des zweiten Anschlusskörpers aufliegt, wobei die Auflagefläche radial von der zentralen Bohrung abzweigende Kanäle aufweist, die im zusammengesetzten Zustand der Vorrichtung die Kapillaren bilden. Der Hülsenkörper mit den Kanälen in der Auflagefläche ist so angeordnet, dass die einzelnen Kanäle jeweils vorzugweise zu einem der Abfuhrvolumen führen. Die zentrale Bohrung der Vorrichtung setzt sich in der zentralen Bohrung des Hülsenkörpers fort.

Die Grundfläche des zweiten Anschlusskörpers weist vorzugsweise einen Sitz für einen scheibenförmigen hydrophoben porösen Bakterienfilter auf. Solch ein Bakterienfilter ist so porös, dass er den Messgasstrom in der Gasprobenleitung oder in der Vorrichtung nicht wesentlich stört, jedoch im Messgasstrom enthaltene Mikroorganismen, beispielsweise Bakterien oder Viren und andere Verunreinigungen zuverlässig vom Gasanalysator fernhält.

Vorzugsweise weist die zentrale Bohrung einlassseitig und/oder auslassseitig Aufnahmen zum Aufstecken oder Einstecken von einem Schlauch oder zwei Schläuchen einer Gasprobenleitung auf. Auf diese Weise kann die erfindungsgemäße Vorrichtung in eine Gasprobenleitung, die ein oder zwei Schläuche umfasst, integriert werden. Es ist auch möglich, einen Schlauch durchzuschneiden und die beiden Schnittenden mit den einlassseitigen und auslassseitigen Aufnahmen der Vorrichtung zu verbinden.

Die Innenbohrung bzw. zentrale Bohrung der Vorrichtung weist vorzugsweise den gleichen Innendurchmesser wie die verwendeten Schläuche der Gasprobenleitung auf.

Die der Erfindung zugrunde liegende Aufgabe wird auch durch eine Gasprobenleitung, umfassend wenigstens einen Schlauch zur Leitung eines zu analysierenden Gases, insbesondere Atemluft, gelöst, bei der zur Vortrocknung eines Gasstroms eine zuvor beschriebene erfindungsgemäße Vorrichtung mit dem Schlauch verbunden ist.

In der Gasprobenleitung ist die Vorrichtung zur Vortrocknung des Gasstroms an einer beliebigen Stelle in der Gasprobenleitung angeordnet.

Schließlich wird die der Erfindung zugrunde liegende Aufgabe durch einen Gasanalysator, insbesondere Kapnograph, mit einer zuvor beschriebenen erfindungsgemäßen Gasprobenleitung und/oder einer zuvor beschriebenen erfindungsgemäßen Vorrichtung zur Vortrocknung eines Gasstroms gelöst.

Sowohl die erfindungsgemäße Gasprobenleitung mit der Vorrichtung als auch der Gasanalysator mit der Gasprobenleitung und/oder der Vorrichtung können länger ununterbrochen betrieben werden als dies bislang der Fall war, da kondensierte Feuchtigkeit in der Vorrichtung zur Vortrocknung effektiv abgeführt und nach außen geführt wird. Da die Abführung über einen Kapillareffekt erfolgt, bildet das Volumen des Ringspalts oder der Abfuhrvolumen keinen Teil des Volumens der Gasprobenleitung selbst, so dass dies kein Totvolumen hinzufügt. Die Flankensteilheit und Genauigkeit der Messung ist somit auch bei hohen Respirationsraten und geringem Absaugvolumen gewährleistet. Da das Wasser nicht aufgefangen wird, sondern nach außen abgeleitet wird, muss die Messung nicht unterbrochen werden, da sich kein Reservoir mit Kondenswasser füllen kann.

Die zu den einzelnen Erfindungsgeständen, also der Vorrichtung, der Gasprobenleitung und dem Gasanalysator vorher genannten Merkmale, Vorteile und Eigenschaften gelten jeweils auch für die anderen Erfindungsgegenstände, auf die sie sich beziehen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine perspektivische schematische Darstellung einer erfindungsgemäßen Vorrichtung zur Vortrocknung eines Gasstroms,
- Fig. 2: eine Seitenansicht und eine Querschnittsdarstellung durch eine Vorrichtung gemäß Fig. 1,
- Fig. 3: schematische Explosionsdarstellungen der Vorrichtung gemäß Fig. 1,
- Fig. 4: perspektivische Darstellungen einer weiteren erfindungsgemäßen Vorrichtung zur Vortrocknung eines Gasstroms,
- Fig. 5: Querschnitts- und Detaildarstellungen der Vorrichtung gemäß Fig. 4 und
- Fig. 6: schematische Explosionszeichnungen der Vorrichtung gemäß Fig. 4.

In den Zeichnungen sind jeweils gleiche oder gleichartige Elemente und/oder Teile mit denselben Bezugsziffern versehen, so dass von einer erneuten Vorstellung jeweils abgesehen wird.

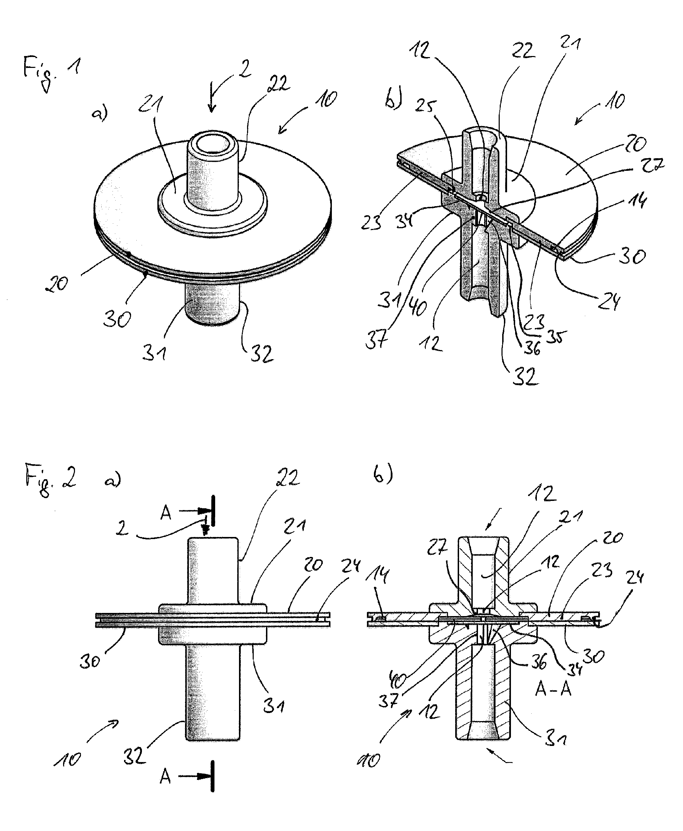

In Fig. 1a) ist ein erstes Ausführungsbeispiel einer erfindungsgemäßen Vorrichtung 10 zur Vortrocknung eines Gasstroms dargestellt. Der Gasstrom strömt in einer mit einem Pfeil dargestellten Gasströmungsrichtung 2 durch eine Einlassaufnahme 22 ein und tritt aus einer Auslassaufnahme 32 wieder aus. An die Einlassaufnahme 22 und die Auslassaufnahme 32 sind Schläuche einer Gasprobenleitung (nicht dargestellt) anschließbar. Diese können beispielsweise in die Aufnahmen 22, 32 eingesteckt werden.

Die Aufnahmen 22, 32 sind jeweils Teil eines ersten Anschlusskörpers 21 und eines zweiten Anschlusskörpers 31. Außerdem weist die Vorrichtung 10 einen ersten Flächenkörper 20 und zweiten Flächenkörper 30 mit rundem Querschnitt auf, die zusammen eine Scheibe bilden.

In Fig. 1b) ist die Vorrichtung 10 im Querschnitt perspektivisch dargestellt. Der erste Flächenkörper 20 weist eine zentrale Aussparung 25 auf, in die der erste Anschlusskörper 21 mit der Einlassaufnahme 22 eingreift. An seinem Umfang weist der erste Flächenkörper 20 eine umlaufende Dichtung 24 auf. Gegenüber dem ersten Flächenkörper 20 ist der zweite Flächenkörper 30 mit ebenfalls rundem Querschnitt mit zentraler Aussparung 35 angeordnet, in die der zweite Anschlusskörper 31 eingesteckt ist. Der Rand ist durch die umlaufende Dichtung 24 abgedichtet. Zwischen dem ersten Flächenkörper 20 und dem zweiten Flächenkörper 30 bleibt ein Ringspalt 14 geöffnet.

In der Fig. 1b) sind ferner vom ersten Flächenkörper 20 ausgehende Abstandhalter 23 sichtbar, die den Abstand zwischen den beiden Flächenkörpern 20, 30 bewahren, so dass der Ringspalt 14 großflächig offenbleibt bis auf die Position der Abstandhalter 23 selber. Außer bei den streifenförmigen Abstandhaltern 23 weist der erste Flächenkörper 20 die gleiche Dicke auf wie der Flächenkörper 30.

Die Einlassaufnahme 22 weist einen Innendurchmesser auf, der dem Außendurchmesser eines einzusteckenden Schlauches entspricht. Er endet in einem als Anschlag ausgebildeten verengten Abschnitt mit einer Zentralbohrung 12, die den gleichen Innendurchmesser aufweist wie ein Innendurchmesser eines Schlauchs einer Gasprobenleitung. Das Gleiche gilt für den zweiten Anschlusskörper 31 und der Auslassaufnahme 32.

Die innere Öffnung des zweiten Anschlusskörpers 31 ist im Bereich des Spaltes 14 trichterförmig erweitert, um einen verwirbelungsfreien Gasstrom nach Durchtritt durch einen hydrophoben Bakterienfilter 40 zu gewährleisten. Mit dem Bezugszeichen 37 ist eine Stützrippe innerhalb des Trichters 36 gekennzeichnet. Es sind drei Stützrippen 37 innerhalb des Trichters 36 umfänglich vorgesehen, um den Bakterienfilter 40 zu stützen.

Auch der erste Anschlusskörper 21 weist an seiner dem Bakterienfilter 40 gegenüberliegenden Seite eine leichte Einwölbung 27 aus, mit der der Abstand zum Bakterienfilter 40 etwas vergrößert ist. Damit wird verhindert, dass sich der Bakterienfilter 40 gegebenenfalls an die gegenüberliegende Fläche des ersten Anschlusskörpers 21 ansaugt.

In der Fig. 2a) ist die Vorrichtung 10 aus Fig. 1 von der Seite perspektivisch dargestellt. Die einfache Außenansicht und der einfache Aufbau werden hieraus deutlich. Von oben nach unten in Gasströmungsrichtung 2 gelangt das Messgas zunächst in die Einlassaufnahme 22 des ersten Anschlusskörpers 21, sodann in den Spalt zwischen erstem Flächenkörper 20 und zweitem Flächenkörper 30, der umfänglich durch die umlaufende Dichtung 24 abgedichtet ist. Anschließend dringt der Gasstrom durch die Auslassaufnahme 32 des zweiten Anschlusskörpers 31 wieder heraus.

In Fig. 2b) ist die gleiche Ansicht im Querschnitt schematisch dargestellt. Dieser Querschnitt geht wiederum durch zwei der Stützkörper 23, so dass der Spalt 14 nicht, wie an den meisten anderen Stellen in Umfangsrichtung, offen dargestellt ist. Gut zu erkennen ist auch der Sitz 34 für den hydrophoben Bakterienfilter 40 im zweiten Anschlusskörper 31.

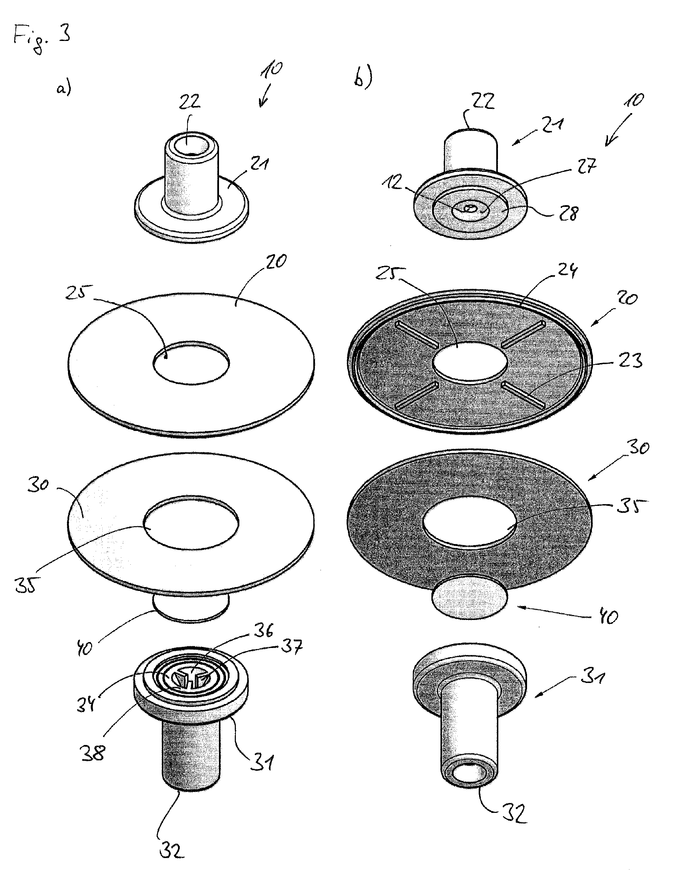

In Fig. 3a) ist eine perspektivische Explosionsdarstellung der Komponenten der erfindungsgemäßen Vorrichtung 10 gemäß Fig. 1 aus einer Blickrichtung von oben, d.h. von der Seite des ersten Flächenkörpers 20 aus gesehen, dargestellt. Der erste Anschlusskörper 21 mit der Einlassaufnahme 22 fluchtet mit der zentralen Aussparung 25 des ersten Flächenkörpers 20. In sehr ähnlicher Geometrie hat der zweite Flächenkörper 30 den gleichen Außenumfang wie der erste Flächenkörper 20. Seine zentrale Aussparung 35 hat einen etwas größeren Durchmesser als die zentrale Aussparung 25 des ersten Flächenkörpers 20. In dieser zentralen Aussparung 35 des zweiten Anschlusskörpers 31 ist dann der Bakterienfilter 40 angeordnet, der hierfür auf einem Sitz 34 mit Schweißringen (ohne Bezugszeichen) auf der Grundfläche 38 des zweiten Anschlusskörpers 31 angeordnet und angeschweißt wird. In dem Sitz 34 überdeckt der Bakterienfilter 40 damit den sich in Gasströmungsrichtung 2 schließenden Trichter 36 mit den drei Stützrippen 37. Der zweite Anschlusskörper 31 mündet in die Auslassaufnahme 32.

In Fig. 3b) ist die gleiche Abfolge in einer perspektivischen Explosionsansicht von unten, d.h. aus der Richtung des zweiten Anschlusskörpers 31, gezeigt. An der Unterseite des ersten Anschlusskörpers 21 ist die ringförmige Grundfläche 28 zu erkennen, deren Außendurchmesser mit dem Innendurchmesser der zentralen Aussparung 25 des ersten Flächenkörpers 20 übereinstimmt. In ihrer Mitte rund um die zentrale Bohrung 12 weist die Grundfläche 28 eine Einwölbung 27 auf.

Der erste Flächenkörper 20 weist an seiner Unterseite Konturierungen auf, nämlich einerseits die umlaufende Dichtung 24 und andererseits vier stabförmige Abstandhalter 23, die in Fig. 1b) und Fig. 2b) jeweils im Querschnitt dargestellt waren.

Der zweite Flächenkörper 30 ist nicht weiter konturiert. Auch der zweite Anschlusskörper 31 weist an seiner Unterseite keine weiteren Konturierungen auf.

Die in den Fig. 3a) und 3b) gezeigten Komponenten werden miteinander verklebt oder verschweißt.

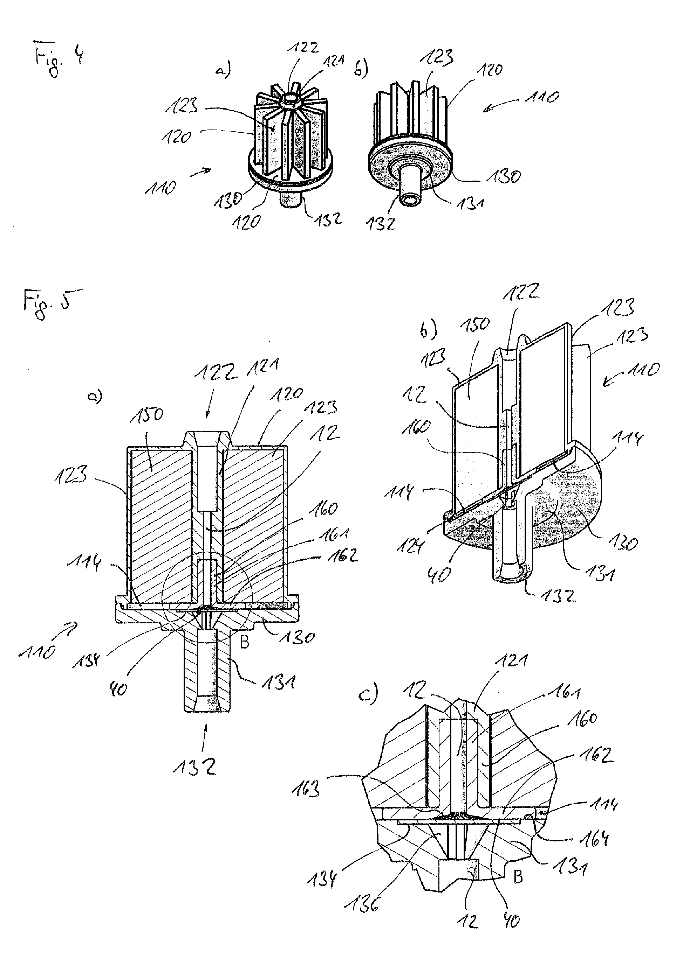

In Fig. 4a) und 4b) ist von zwei unterschiedlichen Blickrichtungen schematisch ein zweites Ausführungsbeispiel einer erfindungsgemäßen Vorrichtung 110 perspektivisch dargestellt. Diese Ausführungsform weist eine Struktur aus radial abstehenden Abdampfrippen 123 oder Abdampfvolumen auf. An ihrer Eingangsseite weist die Vorrichtung 110 eine Einlassaufnahme 122 auf, in die Messgas einströmt. Der Einlassaufnahme 122 ist Teil eines ersten Anschlusskörpers 121. Die Abdampfrippen 123 sind Teil des ersten Flächenkörpers 120. Auch die Grundfläche, von der aus die Abdampfrippen 123 ausgehen, ist ein Teil des ersten Flächenkörpers 120.

Demgegenüber sind auf der Unterseite der zweite Flächenkörper 130 sowie der Anschlusskörper 131 mit der Auslassaufnahme 132. Die Einlassaufnahme 122 und die Auslassaufnahme 132 sind im Prinzip gleichartig ausgestaltet wie die Einlassaufnahme 22 und die Auslassaufnahme 32 aus dem ersten Ausführungsbeispiel gemäß den Fig. 1 bis 3.

Die Abdampfrippenstruktur des Ausführungsbeispiels der Vorrichtung 110 gemäß Fig. 4 bietet eine große Oberfläche eines Materials mit hoher Wasserdampftransportrate, so dass Wasser, das durch den Kapillareffekt aus dem Gasstrom entfernt wurde, mit hoher Effizienz und hoher Durchsetzrate an die Außenluft bzw. Umgebungsluft abgegeben werden kann.

In Fig. 5 sind in den Teilfiguren a), b) und c) Querschnittsdarstellungen und Detaildarstellungen der Vorrichtung 110 gemäß Fig. 4 dargestellt. In Fig. 5a) ist dargestellt, dass die Einlassaufnahme 122 in eine zentrale Bohrung 12 mündet, die zunächst im ersten Anschlusskörper 121 ausgebildet ist. Der erste Anschlusskörper 121 ist einstückig mit dem ersten Flächenkörper 120 ausgebildet, der die Abdampfrippen 123 umfasst. Dieses Ausführungsbeispiel ist somit ein Beispiel eines einstückig ausgebildeten ersten Flächenkörpers 120 und Anschlusskörpers 121.

Innerhalb des Hohlraums der Abdampfrippe 123 ist ein poröser Kapillarkörper 150 dargestellt, der durch seine Porosität die Flüssigkeit bzw. das Kondenswasser, das durch Kapillarkräfte aus dem Gasstrom entfernt worden ist, wiederum durch Kapillarkräfte großräumig innerhalb des Abdampfvolumens verteilt und mit den Wänden aus Material mit hoher Wasserdampftransportrate in Kontakt bringt.

An ihrem unteren Ende enden sowohl der poröse Kapillarkörper 150 als auch der erste Flächenkörper 120 an einem Spalt 114. Dieser kann eine Breite haben, die größer ist als ein eigentlicher Kapillarspalt. Der Spalt wird auf der anderen Seite begrenzt durch den zweiten Flächenkörper 130, der einstückig mit dem zweiten Anschlusskörper 131 und der Auslassaufnahme 132 ausgebildet ist. In seinem zentralen Bereich weist der zweite Flächenkörper 130 also noch einen Sitz 134 für einen Bakterienfilter 40 auf.

Neben dem ersten Flächenkörper und Anschlusskörper 120, 121 sowie dem zweiten Flächenkörper und Anschlusskörper 130, 131 ist auch noch ein Hülsenkörper 160 vorhanden, der einen zylindrischen Teil 161 aufweist, der in eine entsprechende zylindrische Bohrung größeren Durchmessers im ersten Anschlusskörper 131 eingesetzt ist und die zentrale Bohrung 12 mit gleichem Durchmesser fortsetzt. Der Hülsenkörper 160 endet in einer Scheibe 162 mit größerem Radius, die in dem Spalt 114 angeordnet ist und teilweise mit dem porösen Kapillarkörper 150 in Kontakt ist. Auf der Seite des Hülsenkörpers 160, die mit dem zweiten Flächenkörper 130 in Kontakt steht, ist die Oberfläche bzw. die Kontaktfläche des Hülsenkörpers 160 konturiert. Sie weist an dieser Stelle einerseits eine Einwölbung 127 auf wie auch der erste Flächenkörper 20 aus dem Ausführungsbeispiel gemäß Fig. 1 bis 3, andererseits weist der Hülsenkörper 160 an dieser Fläche Einbuchtungen, nämlich Kapillarkanäle 163, auf, die jeweils vom Zentrum nach außen zu den Abdampfrippen 123 führen.

In Fig. 5b) ist der gleiche Schnitt noch einmal in einer perspektivischen Darstellung dargestellt, aus dem sich die geometrische Anordnung noch einmal deutlich ergibt. Auch hier mündet der erste Flächenkörper 120 an seinem Umfang in einer umlaufenden Dichtung 124. In der perspektivischen Darstellung sind insgesamt drei Abdampfrippen 123 dargestellt, von denen zwei im Querschnitt offen dargestellt sind.

In Fig. 5c) ist eine Detaildarstellung gemäß dem Detail B aus Fig. 5a) dargestellt. Im Zentrum ist der Hülsenkörper 160 mit seinem zylindrischen Teil 161 und dem Scheibenteil 162 sowie der Auflagefläche 164, die, wie dem Zentrum zu entnehmen ist, Kapillarkanäle 163 aufweist. Diese setzen sich bis zum Rand der Auflagefläche 164, d.h. bis zum Spalt 114, fort. Dabei ist in Fig. 5c) der Schnitt durch eine der Wände zwischen den Kapillarkanälen 163 gelegt, so dass der Kapillarkanal 163, der nach links oder rechts führt, nicht ausdrücklich dargestellt ist.

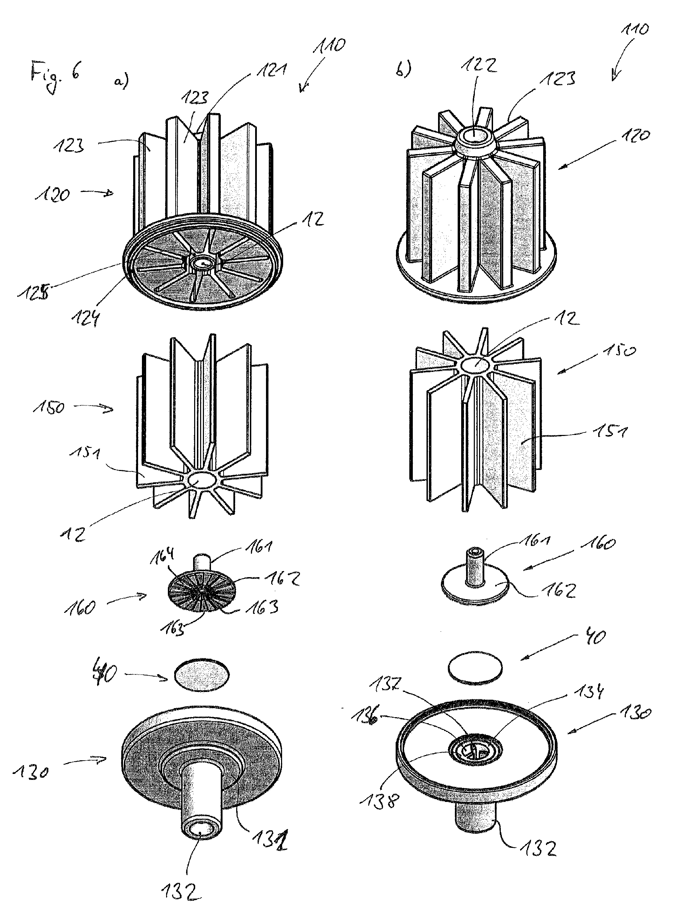

In Fig. 6a) und b) sind schematische perspektivische Explosionsdarstellungen der erfindungsgemäßen Vorrichtung 110 gemäß den Fig. 4 und 5 dargestellt, und zwar in einer Ansicht von unten in Fig. 6a) und in einer Ansicht von oben in Fig. 6b).

Der kombinierte erste Flächenkörper 120 und erste Anschlusskörper 121 ist ein einstückiger Körper aus einem Material mit hoher Wasserdampftransportrate, der beispielsweise in einer Gussform aus einem Kunststoff, beispielsweise einem Polyether-block-amin oder einem Polyether-ester Blockcopolymer herstellbar ist. Er weist auf seiner Oberseite die Struktur der Abdampfrippen 123 auf, die in ihrem Inneren hohl sind, sowie die zentrale zylindrische Struktur mit der zentralen Bohrung 12 und der Einlassaufnahme 122. Diese zentrale zylindrische Struktur ist teilweise durch einen ebenfalls zylindrischen Hohlraum 125 umgeben, von dem die Hohlräume der Abdampfrippen 123 sternförmig abzweigen. An der Unterseite ist dieser Körper flächig mit Ausnahme der Aussparungen für die Hohlräume der Abdampfrippen 123 und des zylindrischen Hohlraums 125 rund um die zentrale zylindrische Form. Umfänglich ist die zentrale Fläche durch die umlaufende Dichtung 124 abgedichtet.

Unterhalb des ersten Flächenkörpers 120 und ersten Anschlusskörpers 121 ist ein in die Hohlräume 125 und die Hohlräume der Abdampfrippen 123 passender poröser Kapillarkörper 150 dargestellt, der die entsprechende sternförmige Struktur mit zentraler Öffnung 12 und Rippen 151 aufweist, der passgenau in den ersten Flächenkörper 120 und Anschlusskörper 121 einsetzbar ist.

Es schließt sich der Hülsenkörper 160 an, der auf seiner Oberseite, in Fig. 6b) dargestellt, einen zylindrischen Teil 161 und einen ebenen scheibenförmigen Teil 162 aufweist, während seine Unterseite, in Fig. 6a) dargestellt, radial auslaufend konturiert ist. Die Auflagefläche 164 weist insgesamt zehn radial verlaufende Vertiefungen auf, die als Kapillarkanäle 163 ausgebildet sind. Der zylindrische Teil 161 wird in einen entsprechend erweiterten zylindrischen Hohlteil des zentralen Zylinders des ersten Anschlusskörpers 121 eingesteckt.

Darunter schließt sich der hydrophobe scheibenförmige Bakterienfilter 40 an, der auf eine entsprechende Auflagefläche bzw. einem Sitz 134 des zweiten Anschlusskörpers 131 aufgesetzt wird und dort mit Schweißringen verschweißt wird. Die Struktur aus Trichter 136, Stützrippen 137 und Grundfläche 138 ist in Fig. 6b) sichtbar. Auch der zweite Flächenkörper 130 und der zweite Anschlusskörper 131 bilden in diesem Ausführungsbeispiel eine Einheit, sind also einstückig ausgebildet.

Der zweite Flächenkörper 130 weist umfänglich ebenfalls einen Rand auf, der zusammen mit der umlaufenden Dichtung 124 an der Unterseite des ersten Flächenkörpers 120 eine Abdichtung des dazwischen offenbleibenden Spaltes 114 bildet.

Auch die in Fig. 6a) und 6b) gezeigten Komponenten werden miteinander verklebt oder verschweißt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 2: Gasströmungsrichtung
- 10: Vortrocknungsvorrichtung
- 12: zentrale Bohrung
- 14: Ringspalt
- 20: erster Flächenkörper
- 21: erster Anschlusskörper
- 22: Einlassaufnahme
- 23: Abstandhalter
- 24: umlaufende Dichtung
- 25: zentrale Aussparung
- 27: Einwölbung
- 28: Grundfläche
- 30: zweiter Flächenkörper
- 31: zweiter Anschlusskörper
- 32: Auslassaufnahme
- 34: Sitz für Mikrobenfilter
- 35: zentrale Aussparung
- 36: Trichter
- 37: Stützrippe
- 38: Grundfläche
- 40: poröser Bakterienfilter
- 110: Vortrocknungsvorrichtung
- 114: Ringspalt
- 120: erster Flächenkörper
- 121: erster Anschlusskörper
- 122: Einlassaufnahme
- 123: Abdampfrippe
- 124: umlaufende Dichtung
- 125: zylindrischer Hohlraum
- 127: Einwölbung
- 130: zweiter Flächenkörper
- 131: zweiter Anschlusskörper
- 132: Auslassaufnahme
- 134: Sitz für Bakterienfilter
- 136: Trichter
- 137: Stützrippe
- 138: Grundfläche
- 150: poröser Kapillarkörper
- 151: Rippe
- 160: Hülsenkörper
- 161: zylindrischer Teil
- 162: Scheibe
- 163: Kapillarkanal
- 164: Auflagefläche

## Patentansprüche

1. Vorrichtung (10, 110) zur Vortrocknung eines Gasstroms mit einer zentralen Bohrung (12) mit einem Einlass und einem Auslass zur Durchleitung eines vorzutrocknenden Gasstroms, insbesondere eines Messgasstroms, wobei die Vorrichtung (10, 110) wenigstens eine Kapillare zur Abführung von Feuchtigkeit aus dem Gasstrom aufweist, die mit der zentralen Bohrung (12) einerseits und mit einer Wand aus einem Material mit hoher Wasserdampftransportrate andererseits in Kontakt steht.

2. Vorrichtung (10, 110) nach Anspruch 1, **dadurch gekennzeichnet, dass** die wenigstens eine Kapillare eine Wandung aus dem Material mit hoher Wasserdampftransportrate aufweist und/oder die Vorrichtung (10, 110) in wesentlichen Teilen aus dem Material mit hoher Wasserdampftransportrate besteht.

3. Vorrichtung (10, 110) nach Anspruch 1 oder 2, **dadurch gekennzeichnet, dass** das Material mit hoher Wasserdampftransportrate ein sulfoniertes Tetrafluorethylen-Polymer, ein Polyether-block-amid Copolymer oder ein Polyether-ester Blockcopolymer umfasst.

4. Vorrichtung (10, 110) nach einem der Ansprüche 1 bis 3, **dadurch gekennzeichnet, dass** die Kapillare als ringförmiger Kapillarspalt (14, 114) ausgebildet ist, der insbesondere eine Ebene aufspannt, die senkrecht auf die zentrale Bohrung steht.

5. Vorrichtung (10, 110) nach einem der Ansprüche 1 bis 4, **dadurch gekennzeichnet, dass** die Vorrichtung (10, 110) einen einlassseitig angeordneten ersten Flächenkörper (20, 120) und einen auslassseitig angeordneten zweiten Flächenkörper (30, 130) aufweist, deren zueinander weisende Grundflächen zueinander kongruent, insbesondere kreisförmig, ausgebildet sind, wobei die Flächenkörper (20, 30, 120, 130) im zusammengesetzten Zustand jeweils am Umfang nach außen abdichtend miteinander kraftschlüssig, materialschlüssig und/oder formschlüssig verbunden sind, wobei zwischen den Grundflächen ein Spalt (14, 114) verbleibt.

6. Vorrichtung (10) nach Anspruch 5, **dadurch gekennzeichnet, dass** der erste Flächenkörper (20) in seinem zentralen Bereich eine Aussparung (25) aufweist und/oder der zweite Flächenkörper (30) in seinem zentralen Bereich eine Aussparung (35) aufweist, wobei in diese Aussparung (25, 35) oder Aussparungen (25, 35) ein erster und/oder zweiter Anschlusskörper (21, 31) eingreift oder eingreifen.

7. Vorrichtung (110) nach Anspruch 5, **dadurch gekennzeichnet, dass** der erste Flächenkörper (120) und/oder der zweite Flächenkörper (130) im zentralen Bereich in einen ersten und/oder zweiten Anschlusskörper (121, 131) übergeht oder übergehen.

8. Vorrichtung (110) nach einem der Ansprüche 5 bis 7, **dadurch gekennzeichnet, dass** der zweite Flächenkörper (130) mehrere radial nach außen gerichtet angeordnete Abfuhrvolumen (123) aufweist, die über den Ringspalt (114) oder jeweils eigene Kapillaren (163) mit der zentralen Bohrung (12) verbunden sind, wobei die Abfuhrvolumen (123) bei kleinem Volumen eine große Oberfläche aus Material mit hoher Wasserdampftransportrate aufweisen.

9. Vorrichtung (110) nach einem der Ansprüche 5 bis 8, **dadurch gekennzeichnet, dass** zwischen dem ersten Flächenkörper (120) und/oder Anschlusskörper (121) und dem zweiten Flächenkörper (130) und/oder Anschlusskörper (131) ein Hülsenkörper (160) mit einer zentralen Bohrung (12) angeordnet ist, der einen zylindrischen Teil (161) und an einem Ende des zylindrischen Teils (161) einen scheibenförmigen Teil (162) aufweist, der im zusammengesetzten Zustand der Vorrichtung (110) mit einer Auflagefläche (164) auf der Grundfläche (138) des zweiten Flächenkörpers (130) und/oder des zweiten Anschlusskörpers (131) aufliegt, wobei die Auflagefläche (164) radial von der zentralen Bohrung (12) abzweigende Kanäle (163) aufweist, die im zusammengesetzten Zustand der Vorrichtung (110) die Kapillaren bilden.

10. Vorrichtung (10, 110) nach einem der Ansprüche 5 bis 9, **dadurch gekennzeichnet, dass** die Grundfläche (38, 138) des zweiten Anschlusskörpers (31, 131) einen Sitz für einen scheibenförmigen hydrophoben porösen Bakterienfilter (40) aufweist.

11. Vorrichtung (10, 110) nach einem der Ansprüche 1 bis 10, **dadurch gekennzeichnet, dass** die zentrale Bohrung (12) einlassseitig und/oder auslassseitig Aufnahmen (22, 32, 122, 132) zum Aufstecken oder Einstecken von einem Schlauch oder zwei Schläuchen einer Gasprobenleitung aufweist.

12. Gasprobenleitung, umfassend wenigstens einen Schlauch zur Leitung eines zu analysierenden Gases, insbesondere Atemluft, **dadurch gekennzeichnet, dass** zur Vortrocknung eines Gasstroms eine Vorrichtung (10, 110) nach einem der Ansprüche 1 bis 11 mit dem Schlauch verbunden ist.

13. Gasanalysator, insbesondere Kapnograph, mit einer Gasprobenleitung nach Anspruch 12 und/oder einer Vorrichtung (10, 110) zur Vortrocknung eines Gasstroms nach einem der Ansprüche 1 bis 11.
